# EUROPEAN PATENT SPECIFICATION

(11) **EP 2 879 743 B1**
(45) Date of publication and mention of the grant of the patent: **07.12.2016**
(21) Application number: 13740036.2
(22) Date of filing: 25.07.2013
(51) Int. Cl.: A61M 13/00, A61M 15/00

(54) **ARRANGEMENT FOR A DRUG DELIVERY DEVICE**
ANORDNUNG FÜR EINE ARZNEIVERABREICHUNGSVORRICHTUNG
AGENCEMENT POUR DISPOSITIF DÝADMINISTRATION DE MÉDICAMENTS

(30) Priority: 31.07.2012 EP 12178704
(43) Date of publication of application: 10.06.2015
(73) Proprietor: Sanofi SA, 1214 Vernier (CH)
(72) Inventor: MAYER, Stefan, 79098 Freiburg Im Breisgau (DE)
(74) Representative: Epping - Hermann - Fischer
(86) International application number: PCT/EP2013/065747
(87) International publication number: WO 2014/019940

(56) References cited:
- WO-A1-02/26299
- WO-A1-2004/033009
- US-A- 3 046 983
- US-A- 4 095 596
- US-B1- 6 240 918

## Description

The present disclosure relates to an arrangement for a drug delivery device, e.g. an inhaler, such as a dry powder inhaler.

Such drug delivery devices are known from US 6,240,918 B1, US 4,095,596 A, WO 2004/033009 A1, DE 10 2007 056 263 A1 and EP 2 201 977 A1, for example.

It is an object of the present disclosure to provide an arrangement suitable for providing an improved drug delivery device. Particularly, an arrangement of components should be provided which facilitates the opening of drug delivery devices.

This object is achieved by the subject-matter of the independent claim. Advantageous embodiments and refinements are subject matter of the dependent claims.

One aspect of the present disclosure relates to an arrangement for a drug delivery device. The arrangement comprises a cap defining a cavity and a body, wherein one of the cap and the body comprises a recess. The other one of the cap and the body is provided with a seal. The seal further comprises a sealing surface. The cap and the body are configured such that the cap is removably attachable to the body. When the cap is attached, the sealing surface of the seal seals a section of the cavity. When the cap comprises the recess, the recess is arranged on a side of the seal which is remote from the section of the cavity. When the body comprises the recess, the seal is arranged on a side of the recess which is remote from the section of the cavity. When the cap comprises the recess and the cap is detached from the body or during the detachment of the cap from the body, the recess is moved towards the side of the seal which faces the section of the cavity and when at least a part of the recess has passed the sealing surface, this part of the recess establishes a passageway between the cavity and the environment. When the cap is provided with the seal and the cap is detached from the body, the seal is moved towards the side of the recess which faces the section of the cavity and when the sealing surface has passed at least a part of the recess, this part of the recess establishes a passageway between the cavity and the environment.

In this way, the passageway may effect a release of the sealing of the section of the cavity which sealing is formed by the sealing surface of the seal cooperating with the body or the cap, respectively. Particularly, the passageway may be a fluid or gaseous passageway, such as an air passageway.

An advantage of the present disclosure is that a force necessary for detaching the cap from the body against the action of an underpressure which may be generated in the sealed section during an opening or detaching movement may be reduced. Particularly, especially the recess may be arranged and configured such that said force is reduced.

In an embodiment, a drug delivery device comprises the arrangement. The body may comprise a drug reservoir with a movable wall. The device may further be an inhaler. The cap may be provisioned to protect the body or further components interacting with the body from external influences, when the device is not in use. The cap may thereby cover parts of the body or openings thereof, when the cap is attached. The cap may further be configured to separate a dose of drug from the reservoir, during an opening or detaching movement. The cap and the body may constitute housing parts of the device, e.g. the cap and the body may constitute the only external parts of the device and/or parts thereof which retain a plurality of inner components of the device. The cap and the body may each comprise a plurality of further components. Furthermore, the cap and the body may form or partly form the device. As an advantage thereof, the device may be sealed from the environment at a defined relative position of the recess, or at least a part thereof, with respect to the seal, when the cap is attached. The arrangement may further aid children or elderly with limited dexterity and/or strength which may have problems in opening such devices against the action of the mentioned underpressure for devices without the recess, as proposed above.

The drug reservoir may retain a dose of drug, preferably a powdery substance, or particularly a multitude of doses thereof. Furthermore, the movable wall may be tracked during use of the device over a multitude of drug dispensing operations. The tracking may be achieved by any suitable mechanism in order to allow for an at least partly homogeneous distribution of drug in the reservoir and thereby for reproducible doses of drug to be dispensed from the device. Particularly, the tracking may be carried out by a biased spring exerting a force on the movable wall of the reservoir, as e.g., a bottom wall of the reservoir. This facilitates provision of reproducible doses, i.e. a dose volume of the doses of drug which are to be dispensed from the reservoir over the lifetime of the device should be as constant as possible.

In an embodiment, at a proximal end, the cap may comprise an open end which provides access to the cavity. The cavity may be configured to partly receive the body, when the cap is attached to the body. The distal end of the cap may be closed.

In an embodiment, the recess is provided in a sidewall of the cap or the body.

In general, the term "recess" may refer to a continuous block-out or clearance in the sidewall of the cap or the body, wherein said block-out or clearance is configured such that a radial passageway between an interior of the cap and the environment may be formed via the recess. In other words, communication between an interior of the cap and the environment is established, when the recess cooporates with the sealing surface. The recess may pierce the respective sidewall.

In an embodiment, the cap comprises the recess and the body is provided with the seal. This is advantageous, as the recess may be fabricated more easily in the sidewall of the cap than in the sidewall of the body.

One embodiment relates to the recess being disposed such that its distance to an open end of the cap is smaller than the distance to a closed end of the cap, particularly when the cap is attached. When the cap comprises the recess, this holds of course also, when the cap is detached.

An advantage of such an arrangement is that the section of the cap which can be sealed, i.e. the section between the recess and the closed end of the cap, when the cap is attached, expediently corresponds to a large volume. Thus, a large part of the device may be sealed in the cavity.

In an embodiment, the cap is configured to be free of any openings or inlets between the recess and the closed end. In other words, the cap is expediently continuous, if applicable except for the recess, if the cap comprises the recess.

In an embodiment, the body comprises at least one opening, which is arranged to establish communication between the environment and an interior of the body, when the cap is detached. When the cap is attached, this opening of the body may be arranged in the section of the cavity. It is also contemplated that the body comprises a plurality of such openings. In the case of an inhaler, e.g. one opening may be provided which allows an air flow to be established such that a user may take in a dose of drug. A further additional or alternative example of such an opening is one which directs an air flow according to a desired flow path layout. An additional or alternative opening may be provisioned in the body in order to allow communication of the environment with a component of the inhaler, e.g., with a dessicator. When the cap is attached at the body, expediently all openings or inlets are sealed.

The term "communication" used herein may refer to, e.g., fluid or gaseous communication, such as air communication which permits fluid and/or gas flow, e.g., between the environment and an interior of the body.

In an embodiment the body comprises a mouthpiece. When the cap is attached, the mouthpiece is expediently arranged in the section of the cavity. The mouthpiece may be comprised or provided on the body and may constitute a distal opening of the body at which the user can apply a suction action in order to take in a dose of drug. The mouthpiece is sealed from the environment and protected against influences therefrom, when the cap is attached.

In an embodiment, the opening is configured to establish communication with an interior region of the body which is arranged outside of the section, the section and the interior region defining a sealed portion, when the cap is attached. An advantage of such a configuration relates to the fact that one or more further components of the device which should be sealed against the environment, when the device is not in use, may then be retained in the body and remote from the section of the cavity or from the cavity. The size of the sealed volume may be increased in this way.

According to an embodiment, no openings are arranged outside of the cap, i.e. there is no communication between the environment and the interior region of the body, when the cap is attached at the body, such that an all-side protection of the one or more further components of the device is guaranteed.

The mentioned components may comprise a desiccator. Desiccators are often used in inhalers to avoid agglutination of powdery substances. Desiccators absorb moisture, e.g., caused by the saliva of the user which uses the device, especially an inhaler. Although desiccators often require comparatively large spaces, there is the need to arrange them in close communication with parts or components interacting with the powdery substance to be dispensed.

According to an embodiment, the interior region defines a sealed portion or a sealed volume delimited by the body and the cap, when the cap is attached. In other words, when the cap is attached at the body, all openings or inlets of the body which could communicate with the environment, when the cap is detached, are enclosed by the cap and, particularly sealed by the seal. As an advantage thereof, an expediently large portion of the device is sealed from the environment and thus protected against harmful external influences, like moisture, for example.

In an embodiment, the detachment of the cap from the body comprises a relative axial movement of the cap and the body. This movement requires a force which has to be exerted by the user in order to overcome an underpressure generated in the section. The recess may be arranged to reduce said force to an extent determined by the axial distance between the sealing surface and the recess, when the cap is attached. In this embodiment, the underpressure may increase with increasing axial distance between the cap and the body while the cap and the body are moved relative to each other until the recess or at least a part thereof establishes the passageway between the cavity and the environment. The relative axial distance is the distance by which the cap and the body have been relatively axially moved during the detachment of the cap. The underpressure generated in the section increases or rather the pressure in the section decreases according to Boyle's law, as the volume of the section is increased, when the cap is being detached.

In an embodiment, the detachment of the cap from the body additionally or alternatively to the axial movement comprises a relative rotation of the cap and the body. The detachment of the cap from the body may relate to the process of opening the device to that effect that the section is unsealed and the mentioned openings of the body may be presented to the user and/or the environment. Accordingly, the attachment of the cap to the body may relate to the process of closing the device, such as after a dose of drug was delivered.

In an embodiment, the arrangement is configured such that, when the cap is detached from the body, a dose of drug is extracted from the reservoir and/or the wall of the reservoir is exposed to the underpressure generated in the sealed section. During detachment, a dose of drug may be separated from the reservoir of the body in which it was retained and moved into a ready-to-dispense-state. When the cap is detached from the body and/or during a relative axial movement of the cap and the body, a dosing hole is pulled out of the reservoir, whereby the dose of drug is extracted from the reservoir. The user may then administer the dose from the ready-to-dispense-state.

The body, particularly a rotation part (see below), may further retain a dosing pin comprising the dosing hole. The dosing hole may be configured to extract drug from the reservoir, e.g. when it is pulled out from or out of the reservoir. When the cap is attached, the dosing hole of the dosing pin may be retained in the reservoir. Movement of the cap may be transferred to the dosing pin to move the dosing hole out of and/or back into the reservoir.

In an embodiment, the wall of the reservoir is exposed to the underpressure in the section of the device, when the cap is being detached. Consequently, the wall could be moved in response to the underpressure during opening of the device, i.e. when the cap is being detached from the body. Particularly, the wall may be retracted. This may reduce dose accuracy and, particularly, result in a wide variation of the sizes of the doses.

The reservoir may be arranged in the sealed portion, at least partly, outside of a region which is enclosed by the section, when the cap is attached.

It is an advantage of the proposed arrangement that the arrangement may be designed such that the underpressure which is generated during opening only reaches moderate values, as compared to prior art devices. Thereby, variations of the sizes of doses of drug to be delivered may be prevented by the arrangement of the present disclosure, as the wall of the reservoir is then only exposed to moderate underpressures. To this effect, the wall which may be spring biased may not be undesirably moved during opening or only to a reduced extent, i.e. the wall may, for example, not be retracted significantly.

In an embodiment the body comprises a base part and a rotation part, wherein said parts are rotatably connected to each other.

In an embodiment, when the seal is provided on the body, the seal is provided on the base part of the body. The seal may be provided between a connection element, e.g., a thread element, on the base part and a gripping section of the base part. The gripping section is a section which may be gripped by the user when the cap is attached. Accordingly, the gripping section is expediently exposed to the environment, when the cap is attached.

In an embodiment the rotation part is arranged in the section, when the cap is attached.

One or more openings or inlets of the body, as, e.g. the mouthpiece or openings which direct an air flow according to the desired flow path layout, are expediently disposed on the rotation part of the body. When the seal is provided on the body, the seal is further preferably provided on the base part. The cap preferably interacts with the rotation part to rotate this part, when the cap is being detached from the body.

In an embodiment, the recess is delimited by at least two side faces with different inclinations with respect to a main axis of extent of the one of the cap and the body comprising the recess. In other words, the side faces may define different angles with respect to a main axis of extent.

Preferably, the main axis of extent is the longitudinal axis of the cap, the body and/or of the device.

Preferably, the recess is delimited by two side faces, wherein one of the side faces defines a smaller angle and the other one defines a greater angle with the longitudinal axis.

In an advantageous embodiment, when the cap comprises the recess, the recess may be disposed at a proximal end face of the cap. Accordingly, the recess may constitute a cut-out, expediently an axially extending cut-out of the proximal end face. Alternatively, the recess may be disposed remote from the end face of the cap such that it is completely surrounded by material of the sidewall of the cap, e.g. a hole in the sidewall, preferably nearer at the open end than at the closed end.

According to the previous embodiment, the recess may comprise a cut-out at the end face of the cap, wherein the side faces of the recess define a tooth-like shape of the recess. According to this embodiment, the recess comprises an inner edge which delimits the inner surface of the cap. The inner edge is provided with a chamfer which interacts with the sealing surface of the seal, when the cap is attached to the body. The chamfer further aids the attachment of the cap to the body, as it provides a smoother contact with the sealing surface.

In an embodiment the attachment of the cap to the body comprises a rotational closing movement of the cap with respect to the body in a closing direction of rotation and the side face which defines a smaller angle with respect to the main axis of extent forms a rotational stop face which is configured to block further closing rotation of the cap relative to the body by an interaction of the rotational stop face with a corresponding face on the other one of the cap and the body, when the cap is attached to the body. As an advantage, the corresponding face on the one of the cap and the body not comprising the recess, may act as a counterstop for the rotational stop face of the recess, when the cap is attached to the body.

In a preferred embodiment, the cap may be releasably attached to the body via a thread-connection, e.g. whereby a thread connection element of the cap may be provided at the inner surface of the cap and a thread connection element of the body is provided at an outer surface of the body, preferably an outer surface of the base part.

Thus the cap may be attached at the body at a defined position determined by the rotational stop face of the recess and the corresponding face. Thereby, the user is given feed-back that the cap has been completely attached to the body and, e.g., an overwinding of the threads is prevented, which would likely occur if axial stop faces were used.

Preferably, the one of the side faces of the recess comprising the rotational stop face may be aligned axially, i.e. aligned along or parallel to the longitudinal axis. In this way, a rotational stop of the cap and the body is formed most expediently. The other one or another one of the side faces may be aligned or almost aligned with windings defining the pitch of the thread connection of the cap and the body. That is to say, said windings and said other side face define the same or almost the same angle with the longitudinal axis.

An advantageous embodiment relates to the detachment of the cap from the body comprising a rotational opening movement of the cap relative to the body in an opening direction of rotation, wherein, when the cap is attached, the recess is arranged such that the passageway is established within an angle of rotation of less than 360°, when the cap is rotated relative to the body in the opening direction of rotation. According to this embodiment, the passageway is preferably established within an angle of rotation of less than 180°, most preferably less than 90°. As an advantage thereof, the underpressure which is generated in the section and the force necessary for the opening of the device to overcome the said underpressure is expediently kept small.

In an embodiment one of the cap and the body comprises a plurality of recesses, the recesses being preferably disposed circumferentially. The recesses are further preferably disposed at the same axial position such that also the rotational stop faces of the recesses are arranged at the same axial position.

Accordingly, also a plurality of rotational stop faces of the body may be provisioned circumferentially disposed along the body. Furthermore, also a plurality of passageways between the cavity and the environment may be established simultaneously, one by way of each recess, when during detachment of the cap from the body at least parts of the recesses have passed the sealing surface.

In an embodiment, the recesses are arranged such that adjacent side faces of different adjacent recesses have different inclinations with respect to a main axis of extent of the one of the cap and the body comprising the recesses.

These arrangements provide advantageously a greater mutual stability of the cap and the body due to a plurality of rotational stops, when the cap is attached to the body.

Features which are described herein above and below in conjunction with different aspects or embodiments may also apply for other aspects and embodiments.

Further features and advantages of the subject matter of this disclosure will become apparent from the following description of the exemplary embodiment in conjunction with the figures, in which:

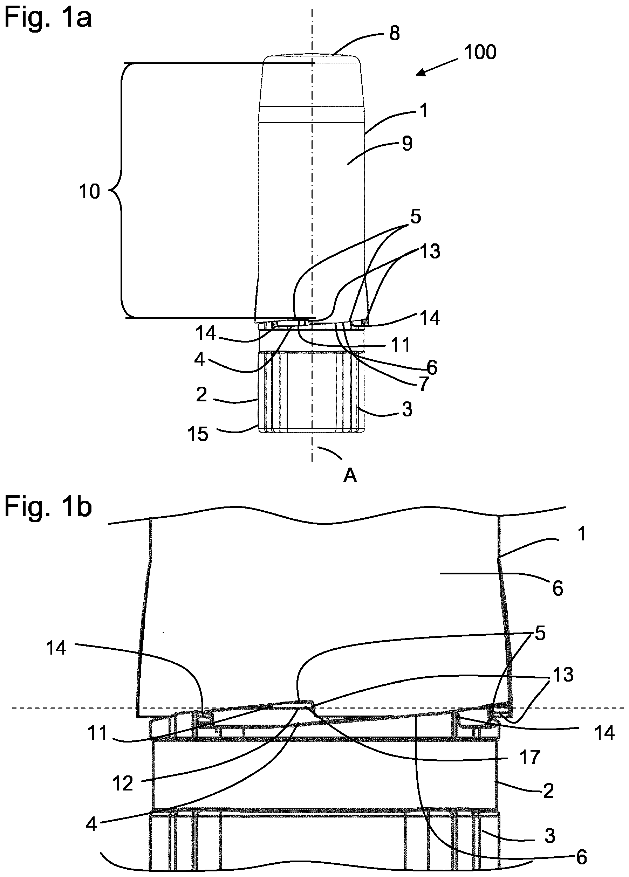

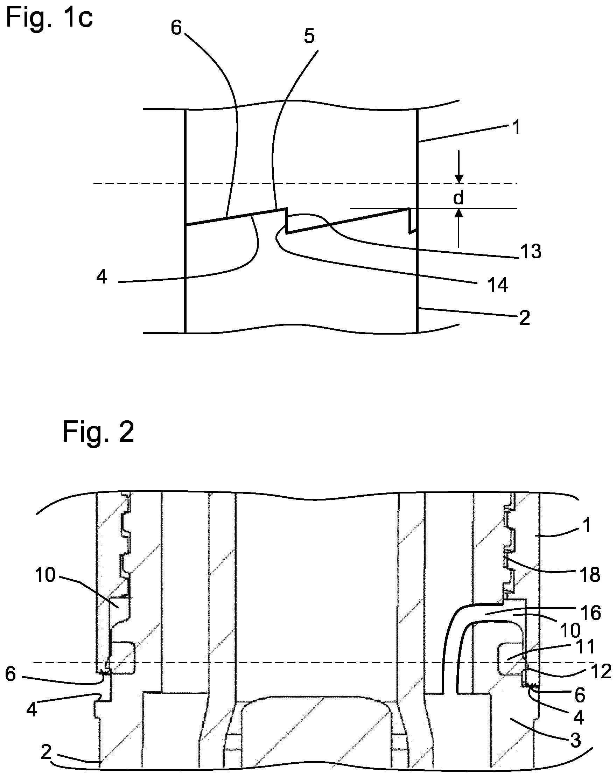

Figure 1 a shows an exemplary embodiment of an arrangement according to the present disclosure, by way of a drug delivery device comprising the arrangement.
Figure 1 b shows an enlarged portion of the image of Figure 1 a.
Figure 1 c shows a schematic representation of a fraction of the arrangement according to the present disclosure.
Figure 2 shows a cross-section of an exemplary embodiment of the arrangement according to the present disclosure.
Figure 3 shows an exemplary embodiment of a fraction of a drug delivery device comprising the arrangement according to the present disclosure based on a longitudinal sectional view.
Figure 4 shows an exemplary embodiment of a body of the drug delivery device based on a longitudinal sectional view.

Like elements, elements of the same kind and identically acting elements may be provided with the same reference numerals in the figures. Additionally, the figures may not be true to scale. Rather, certain figures may be depicted in an exaggerated fashion for better illustration of important principles.

Figure 1 a depicts an arrangement 100 comprising a cap 1 and a body 2. The body further comprises a base part 3 with a face 4. The cap comprises a recess 5 provided at a distal end face 6, an open end 7 and a closed end 8. The recess 5 is shaped as a tooth-like cut-out or block-out in the distal end face 6 of cap 1 (see also figure 1 b). Actually, two recesses 5 are depicted in figures 1 a, 1b and 1 c, respectively which recesses are adjacently arranged at the same axial position, but circumferentially spaced. In the embodiment illustrated herein, the cap 1 comprises four recesses 5. Only two recesses 5 are shown in figures 1 a, 1b and 1 c, respectively. However, other numbers of recesses 5 are also possible.

The cap 1 and the body 2 are aligned along a common longitudinal axis A and may be embodied rotationally symmetric or almost rotationally symmetric. The cap 1 further defines a cavity 9 in which the body 2 is partly retained. A section 10 of the cavity 9 is defined by the axial distance d between the recess 5 and the closed wall 8 of the cap 1. The body 2 is provided with a seal 11 which, comprises a sealing surface 12 (see figure 1 b for a more detailed view).

The cap 1 and the body 2 are configured such that the cap 1 is removably attachable to the body 2. The attachment of the cap 1 at the body 2 comprises a relative axial movement of the cap 1 and the body 2, whereby the body 2 may be partly retained in the cavity 9. The attachment of the cap 1 at the body 2 may further comprise a relative rotational movement.

In the figures, the cap 1 is not completely attached to the body 2, such that the seal 11 remains partly visible or accessible. That is to say, a part of the recess 5 is arranged above the seal 11, or rather above the sealing surface 12 (see figure 1 b) of the seal 11. Another portion of the seal 11 which is covered by the cap 1 interacts with an interior surface of the cap 1, thereby already forming a sealing between this portion of the seal 11 and the interior surface of the cap 1. If the cap 1 is completely attached to the body 2, the sealing surface 12 is no longer visible (see figure 1 c).

The recess 5 forms a rotational stop face 13 which constitutes a side face of the recess 5, particularly the side face which is almost aligned vertical. The body 2 comprises a corresponding face 14 abutting rotational stop face 13 when the cap 1 is completely attached at the body 2 (see figure 1 c). Then, the rotational stop face 13 abuts the corresponding face 14. The distal end face 6 of the cap may abut the face 4 of the body 2.

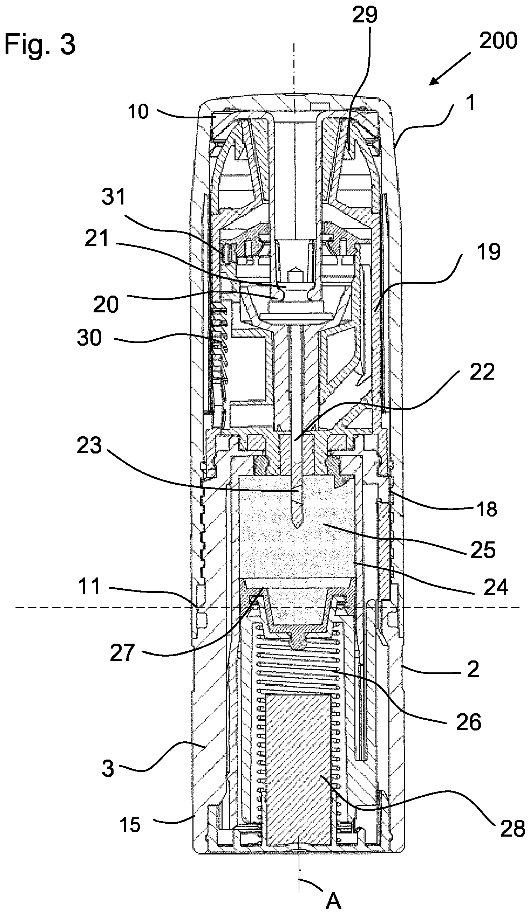

The cap 1 and the body 2 may be part of a drug delivery device 200 (see figure 3), such as an inhaler, like a dry powder inhaler which is closed, when the cap 1 is attached at the body 2. The body 2 further comprises a gripping section 15 which can be gripped by a hand of the user during opening and closing of the device. In order to close the device, the cap 1 and the body 2 have to be axially aligned and relatively axially moved such that a part of the body 2 can be retained in the cavity 9 of the cap 1. Then, cap 1 and body 2 may have to be relatively rotated in a closing direction of rotation.

In order to open the device again, i.e. to detach the cap 1 from the body 2, the cap 1 and the body 2 have to be relatively rotated in an opening direction of rotation opposite to the closing direction. The detachment of the cap 1 from the body 2 also comprises a relative axial and a relative rotational movement of the cap 1 and the body 2.

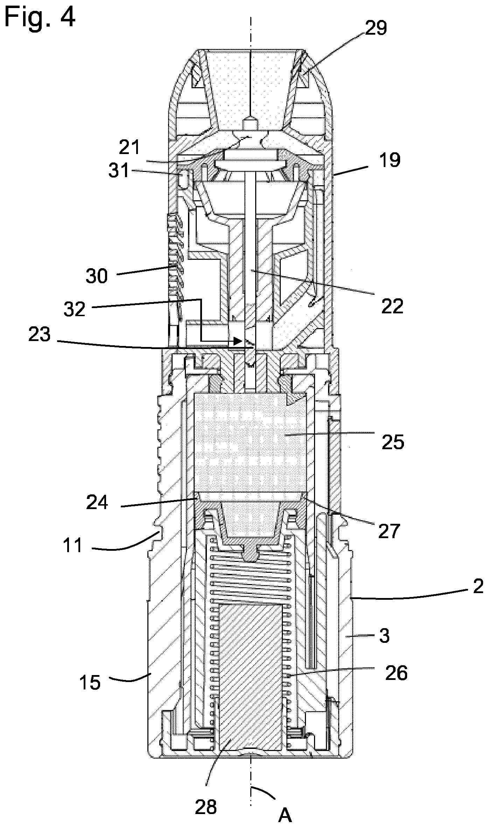

When the cap 1 is attached at the body 2 (see figure 1 c), the section 10 of the cavity 9 is sealed, as any openings (see 16, 29, 30, 31 in the figures 3 and 4) of the body 2 which are arranged to establish communication between the environment and an interior of the body 2, when the cap 1 is detached, are then enclosed by the section 10. That is to say, none of these openings is arranged outside of the section 10. Thus, along with the section 10 of the cavity 9, also the interior of the body 2 or particularly an interior space of the body 2 is sealed against the environment, when the cap 1 is attached. The mentioned openings further establish communication with an interior region of the body which is arranged outside of the section 10 of the cavity 9. For example, the interior region may be arranged in the base part 3 of the body 2, thereby being arranged below the section 10. The section 10 and the interior region form a sealed portion of the device, when the cap 2 is attached. The interior region may thereby communicate with the section 10 via a channel 16 (see figure 2).

Figure 1 b shows a fraction of the image from figure 1 a in the vicinity of the interface between the cap 1 and the body 2 in greater detail. It is apparent that a part of the recess 5, particularly the upper part thereof is arranged above the sealing surface 12 of the seal 11. The seal 11 is provided on the body 2 and arranged horizontally around the circumference of the body 2. The sealing surface 12 actually exhibits the part of the seal 11 which interacts with the inner surface of the cap 1, when the cap 1 is attached at or being attached to the body 2. The seal 11 may, e.g., be an o-ring forming a moisture-tight sealing and/or a bacteria-proof sealing of the section 10 against the environment. A bacteria-proof sealing relates to a gas exchange of the closed device with the environment of less than about 0.33 cm³ per hour.

The plane of the sealing surface 12 is indicated by the dashed horizontal line in figure 2. It is further apparent that the recess 5 is at least partly arranged above the sealing surface 12. Thereby, the recess 5 or an upper part thereof and the seal 11 or rather the sealing surface 12 form a passageway 17. When the cap 1 is attached at the body 2 (see figure 1 c) and the cap 1 is then partly rotated in the opening direction of rotation in order to open the device 200, the passageway 17 is established as soon as at least a part, i.e. the upper part of the recess 5 has passed the sealing surface 12 of the seal 11. Thus, the sealing formed by the sealing surface 12 is released and the passageway 17 thus allows communication between the section 10 and the environment.

When the cap 1 is detached or being detached from the body 2 (see figure 2) an underpressure may be generated in the section 10, as the volume of the section 10 is increased as long as the sealing is tight, when detaching the cap 1. This underpressure increases through relative axial movement of the cap 1 and the body 2, such as during opening of the device 200. This underpressure increases until the passageway 17 is established. As soon as at least a part of the recess 5 is arranged above the sealing surface 12, the underpressure is cleared, as the medium, e.g., the air inside the device 200 or the sealed portion can communicate with the environment by the passageway 17, such that pressure differences are compensated. According to the number of four recesses 5 present in the illustrated embodiment, also four passageways are established at a time, when the cap 1 is detached. In order to overcome the underpressure in the section 10 during opening of the device 200, the user has to manually apply a force which comtinuously increases, when the cap 1 is removed from the body 2.

Figure 1 c shows schematically a portion of the arrangement 100 similar to that of figure 1 b, wherein the cap 1 is attached at the body 2. The sealing surface 12 provided on the body 2 and indicated as a dashed horizontal line is arranged above the recesses 5 which are comprises by the cap 1 such that the section 10 and the interior of the body 2 are sealed against the environment. The recesses 5 shown are arranged to reduce the above mentioned force to an extent determined by the axial distance d (see figure 1 c) between the sealing surface of the seal 11 and the recess 5, when the cap 1 is attached. As compared to a prior art device, the underpressure generated during opening of the device 200 is reduced in this way and also the force necessary to open the device 200.

Figure 2 shows a cross section of the arrangement 100 or the device 200, wherein the section 10 of the cavity 9 is sealed by the seal 11. The sealing surface 12 is again indicated as a dashed horizontal line. Two recesses 5 are visible only as cross sections of the distal end face 6 of the cap 2 on the right and on the left side of the image and both are arranged below the sealing surface 12. Consequently, there is no passageway established and the section 10 of the cavity 9 is sealed. However, the cap 1 is not completely attached at the body 2, as the distal end face 6 does not abut the face 4 on the left hand side of figure 2. Consequently, an underpressure is already generated in the section 10, as the relative axial distance between the cap 1 and the body 2 is increased as compared to the case, when the cap 1 is completely attached.

The cap 2 is thus only partly attached to the body 2 via a thread connection 18. Only the base part 3 of the body 2 is shown in the figure. The base part 3 comprises a channel 16 which is arranged below the thread connection 18 and which establishes communication between the section 10 of the cavity 9 and the interior region of the body 2.

The recesses 5 may be provided with a chamfer (not shown) for a smoother interaction of the recesses 5 or an inner edge of the distal end face 6 of the cap 1 with the sealing surface 12 of seal 11, when the cap 1 is being attached to or detached from the body 2.

Figure 3 shows a longitudinal cross section of a drug delivery device 200 which also comprises the arrangement 100, wherein the cap 1 is attached at the body 2. Thus, the device 200 which may comprise the cap 1 and the body 2 and additional components is closed. The body 2 comprises a rotation part 19 which is rotatable with respect to the base part 3, retained in the cap 2 and enclosed by the section 10. The rotation part 19 is preferably not detachable from the base part 3.

The cap 1 further comprises a cap coupling element 20 and the body further comprises a body coupling element 21, wherein the said coupling elements are engaged. In a state, wherein the cap 1 is not completely attached and/or wherein the section 10 of the cavity 9 is not sealed, the said coupling elements 20, 21 may also be engaged. The body 2, particularly the rotation part 3 may further retain a dosing pin 22 comprising a dosing hole 23. The body 2, particularly the rotation part 3 may further comprise a reservoir 24 which may retain a drug 25. The dosing hole 23 may be configured to extract drug 25 from the reservoir 24, when it is pulled out from the reservoir 24. Particularly, the device 200 may be an inhaler and the reservoir 24 may retain a powdery substance. The dosing pin 22 may be coupled to the body coupling element 21 and, when the cap 1 is attached, the dosing hole 23 of the dosing pin 22 may be retained in the reservoir 24. The body 2, particularly, the base part 3 may be provided with a spring 26, preferably a cylindrical spiral spring which biases a wall 27 of the reservoir 24, particularly a bottom wall thereof. Thereby, the drug 25 or powdery substance may be tracked during operation of the device 200 in order to guarantee that the dosing pin 22 or rather the dosing hole 23 extracts a reproducible or constant volume of drug 25 from the reservoir 24. The tracking may keep the powder compact. The base part 3 of the body 2 further comprises a desiccator 28 which is arranged within a space delimited by the spring 26. The space, where the spring 26 and the desiccator 28 are retained in, is preferably at least partly arranged in the interior region of the body 2 and expediently communicates with the section 10 of the cavity 9 via the channel 16, when the cap 1 is attached, in order to desiccate the sealed portion of the device 200.

The wall 27 of the reservoir 23 may - during opening of the device 200 - be exposed to the underpressure generated in the section 10 and which increases during opening of the device 200. As a consequence, the wall 28 of the reservoir 25 may be retracted, as it is in fluid communication, via channel 16, with the section 10 of the cavity 9, when the cap 1 is attached.

The body 2 further comprises openings, such as a mouthpiece 29 and a first and a second inlet 30, 31 which are expediently arranged on the rotation part 19 of the body 2. In figure 3, these openings are enclosed by the section 10 of the cavity 9 and sealed against the environment.

Figure 4 shows a longitudinal cross section of the body 2. The cap 1 (not shown) is completely detached from the body 2. To this effect, the coupling formed by the cap coupling element 20 and the body coupling element 21 is not established. However, the detachment of the cap 1 from the body 2, particularly, the relative axial movement of the cap 1 and the body 2 have effected that during the detachment of the cap 1, the dosing pin 22 which is coupled to the body coupling element 21 was pulled out of the reservoir 24. The device 200 is now in a ready-to-dispense-state. In this state, the dosing hole 23 of the pin 22 which now contains a dose of drug 25 or powdery substance, is aligned with an air flow path 32 (indicated by the arrow). This air flow may be established by the suction air flow of the user of the device 200 which is applied via the mouthpiece 29.

The first inlet 30 may be provisioned as an air inlet, while the second inlet 31 may aid or direct the air flow or the delivery of drug 25 or powdery substance in a later stage of the drug delivery.

The term "drug" and/or "powdery substance", as used herein may mean a pharmaceutical formulation containing at least one pharmaceutically active compound, for example for the treatment of obstructive airway or lung diseases such as asthma or chronic obstructive pulmonary disease (COPD), local respiratory tract oedema, inflammation, viral, bacterial, mycotic or other infection, allergies, diabetes mellitus.

The active pharmaceutical compound is preferably selected from the group consisting of active pharmaceutical compounds suitable for inhalation, preferably antiallergenic, antihistamine, anti-inflammatory, antitussive agents, bronchodilators, anticholinergic drugs, and combinations thereof.

The active pharmaceutical compound may for example be chosen from:
an insulin such as human insulin, e.g. a recombinant human insulin, or a human insulin analogue or derivative, a glucagon-like peptide (GLP-1) or an analogue or derivative thereof, or exendin-3 or exendin-4 or an analogue or derivative of exendin-3 or exendin-4;
an adrenergic agent such as a short acting β2-agonists (e.g. Salbutamol, Albuterol, Levosalbutamol, Fenoterol, Terbutaline, Pirbuterol, Procaterol, Bitolterol, Rimiterol, Carbuterol, Tulobuterol, Reproterol), a long acting β2-agonist (LABA, e.g. Arformoterol, Bambuterol, Clenbuterol, Formoterol, Salmeterol), an ultra LABA (e.g. Indacaterol) or another adrenergic agent (e.g. Epinephrine, Hexoprenaline, Isoprenaline (Isoproterenol), Orciprenaline (Metaproterenol));
a glucocorticoid (e.g. Beclometasone, Budesonide, Ciclesonide, Fluticasone, Mometasone, Flunisolide, Betamethasone, Triamcinolone);
an anticholinergic agent or muscarinic antagonist (e.g. Ipratropium bromide, Oxitropium bromide, Tiotropium bromide);
a mast cell stabilizer (e.g. Cromoglicate, Nedocromil);
a xanthine derivative (e.g. Doxofylline, Enprofylline, Theobromine, Theophylline, Aminophylline, Choline theophyllinate);
an eicosanoid inhibitor, such as a leukotriene antagonist (e.g. Montelukast, Pranlukast, Zafirlukast), a lipoxygenase inhibitor (e.g. Zileuton) or a thromboxane receptor antagonist (e.g. Ramatroban, Seratrodast);
a phosphodiesterase type-4 inhibitor (e.g. Roflumilast);
an antihistamine (e.g. Loratadine, Desloratadine, Cetirizen, Levocetirizine, Fexofenadine);
an allergen immunotherapy (e.g. Omalizumab);
a mucolytic (e.g. Carbocisteine, Erdosteine, Mecysteine);
an antibiotic or antimycotic;
or a combination of any two, three or more of the above-mentioned compound classes or compounds (e.g. Budesonide/Formoterol, Fluticasone/Salmeterol, Ipratropium bromide/Salbutamol, Mometasone/Formoterol);
or a pharmaceutically acceptable salt or solvate or esters of any of the above named compounds.

Pharmaceutically acceptable salts are for example acid addition salts and basic salts. Acid addition salts are e.g. a chloride, bromide, iodide, nitrate, carbonate, sulfate, methylsulfate, phosphate, acetate, benzoate, benzenesulfonate, fumarate, malonate, tartrate, succinate, citrate, lactate, gluconate, glutamate, edetate, mesylate, pamoate, pantothenate or a hydroxy-naphthoate salt. Basic salts are for example salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology. Pharmaceutically acceptable ester may for example be acetates, propionates, phosphates, succinates or etabonates.

Pharmaceutically acceptable solvates are for example hydrates.

Although the exemplary embodiment was described for an arrangement 100, wherein the cap 1 comprises the recess 5 and the body 2 is provided with the seal 11, the disclosed concept could also be realized with an arrangement 100, wherein the cap is provided with the seal and the recess is provided on the body. Also a hole in the cap 1 is suitable for the recess 5, instead of a cut-out in the distal end face 5. If the recess is provided on the body, the recess is expediently invisible from the outside. When the cap is attached, the sealing surface is expediently arranged axially above the recess.

The scope of protection is not limited to the examples given hereinabove. The invention is embodied in each novel characteristic and each combination of characteristics, which particularly includes every combination of any features which are stated in the claims, even if this feature or this combination of features is not explicitly stated in the claims or in the examples.

### Reference numerals

- 1: Cap
- 2: Body
- 3: Base part
- 4: Face
- 5: Recess
- 6: Distal end face
- 7: Open end
- 8: Closed end
- 9: Cavity
- 10: Section
- 11: Seal
- 12: Sealing surface
- 13: Rotational stop face
- 14: Corresponding face
- 15: Gripping section
- 16: Channel
- 17: Passageway
- 18: Thread connection
- 19: Rotation part
- 20: Cap coupling Element
- 21: Body coupling Element
- 22: Dosing pin
- 23: Dosing hole
- 24: Reservoir
- 25: Drug
- 26: Spring
- 27: Wall
- 28: Desiccator
- 29: Mouthpiece
- 30: First inlet
- 31: Second inlet
- 32: Path
- 100: Arrangement
- 200: Drug delivery device

- d: Axial distance
- A: Longitudinal axis

## Claims

1. An arrangement (100) for a drug delivery device (200), the arrangement (100) comprising:
- a cap (1) defining a cavity (9) and
- a body (2),
- wherein one of the cap (1) and the body (2) comprises a recess (5),
- wherein the other one of the cap (1) and the body (2) is provided with a seal (11), the seal (11) comprising a sealing surface (12),
- wherein the cap (1) and the body (2) are configured such that the cap (1) is removably attachable to the body (2),
- wherein, when the cap (1) is attached, the sealing surface (12) of the seal (11) seals a section (10) of the cavity (9) and wherein,
- A) when the cap (1) comprises the recess (5):
the recess (5) is arranged on a side of the seal (11) which is remote from the section (10) of the cavity (9), during detachment of the cap (1) from the body (2) the recess (5) is moved towards the side of the seal (11) which faces the section (10) of the cavity (9), and, when at least a part of the recess (5) has passed the sealing surface (12), this part of the recess (5) establishes a passageway (17) between the cavity (9) and the environment
- B) when the body (2) comprises the recess (5):
the seal (11) is arranged on a side of the recess (5) which is remote from the section (10) of the cavity (9), during detachment of the cap (1) from the body (2) the seal (11) is moved towards the side of the recess (5) which faces the section (10) of the cavity (9) and, when the sealing surface (12) has passed at least a part of the recess (5), this part of the recess (5) establishes a passageway (17) between the cavity (9) and the environment.

2. The arrangement according to claim 1, wherein the cap (1) comprises the recess (5) and the body (2) is provided with the seal (11).

3. The arrangement according to claim 1 or 2, wherein the recess (5) is disposed such that its distance to an open end (7) of the cap (1) is smaller than the distance to a closed end (8) of the cap (1).

4. The arrangement according to at least one of the previous claims, wherein the body (2) comprises at least one opening (29, 30, 31), which is arranged to establish communication between the environment and an interior of the body (2) when the cap (1) is detached, wherein, when the cap (1) is attached, this opening of the body (2) is arranged in the section (10) of the cavity (9).

5. The arrangement according to claim 4, wherein the opening (29, 30, 31) is configured to establish communication with an interior region of the body (2) which is arranged outside of the section (10), the section (10) and the interior region defining a sealed portion when the cap is attached.

6. The arrangement according to at least one of the previous claims, wherein the detachement of the cap (1) from the body (2) comprises a relativ axial movement of the cap (1) and the body (2) which movement requires a force to be exerted in order to overcome an underpressure generated in the section, and wherein the recess (5) is arranged to reduce said force to an extent determined by the axial distance (d) between the sealing surface (12) and the recess (5), when the cap (1) is attached.

7. The arrangement according to at least one of the previous claims, wherein the recess (5) is delimited by at least two side faces with different inclinations with respect to a main axis of extent of the one of the cap (1) and the body (2) comprising the recess (5).

8. The arrangement according to claim 7, wherein the attachment of the cap (1) to the body (2) comprises a rotational closing movement of the cap (1) with respect to the body (2) in a closing direction of rotation and the side face which defines a smaller angle with respect to the main axis of extent, forms a rotational stop face (13) which is configured to block further closing rotation of the cap (1) relative to the body (2) by an interaction of the rotational stop face (13) with a corresponding face (14) on the other one of the cap (1) and the body (2), when the cap (1) is attached to the body (1).

9. The arrangement according to at least one of the previous claims, wherein the detachment of the cap (1) from the body (2) comprises a rotational opening movement of the cap (1) relative to the body (2) in an opening direction of rotation, and wherein, when the cap (1) is attached, the recess (5) is arranged such that the passageway (17) is established within an angle of rotation of less than 360°, when the cap (1) is rotated relative to the body in the opening direction of rotation.

10. The arrangement according to at least one of the previous claims, wherein the one of the cap (1) and the body (2) comprises a plurality of recesses (5), the recesses being disposed circumferentially.

11. The arrangement according to claim 10, wherein the recesses (5) are arranged such that adjacent side faces of different adjacent recesses have different inclinations with respect to a main axis of extent of the one of the cap (1) and the body (2) comprising the recesses.

12. The arrangement according to at least one of the previous claims, wherein the body (2) comprises a base part (3) and a rotation part (19), wherein said parts are rotatably connected to each other, and wherein, when the seal (11) is provided on the body (2), the seal (11) is provided on the base part (3), and when the cap (1) is attached, the rotation part (19) is arranged in the section (10).

13. The arrangement according to at least one of the previous claims, wherein the body (2) comprises a mouthpiece (29), and wherein, when the cap is attached, the mouthpiece (29) is arranged in the section of the cavity (9).

14. A drug delivery device (200) comprising the arrangement according to at least one of the previous claims, wherein the body (2) comprises a drug reservoir (24) with a movable wall (27), and wherein the device (200) is an inhaler.

15. The drug delivery device (200) according to claim 14, wherein the arrangement is configured such that, during detachment of the cap (1) from the body (2), particularly during a relative axial movement of the cap (1) and the body (2), a dosing hole (23) is pulled out of the reservoir (24), whereby a dose of drug (25) is extracted from the reservoir (24) and the wall (27) of the reservoir (24) is exposed to the underpressure generated in the sealed section (10), wherein a dosing pin comprises the dosing hole, the dosing pin being retained in the body.

## Patentansprüche

1. Anordnung (100) für eine Arzneiverabreichungsvorrichtung (200), wobei die Anordnung (100) Folgendes umfasst:
- eine Kappe (1), die einen Hohlraum (9) definiert, und
- einen Körper (2),
- wobei einer aus der Kappe (1) und dem Körper (2) eine Aussparung (5) umfasst,
- wobei der andere aus der Kappe (1) und dem Körper (2) mit einer Dichtung (11) versehen ist, wobei die Dichtung (11) eine Dichtungsoberfläche (12) umfasst,
- wobei die Kappe (1) und der Körper (2) derart konfiguriert sind, dass die Kappe (1) an dem Körper (2) lösbar anbringbar ist,
- wobei, wenn die Kappe (1) angebracht ist, die Dichtungsoberfläche (12) der Dichtung (11) einen Abschnitt (10) des Hohlraums (9) abdichtet, und wobei
- A) wenn die Kappe (1) die Aussparung (5) umfasst:
die Aussparung (5) auf einer Seite der Dichtung (11) angeordnet ist, die von dem Abschnitt (10) des Hohlraums (9) entfernt ist, während der Entfernung der Kappe (1) von dem Körper (2) die Aussparung (5) zu der Seite der Dichtung (11) bewegt wird, die zu dem Abschnitt (10) des Hohlraums (9) weist, und, wenn mindestens ein Teil der Aussparung (5) die Dichtungsoberfläche (12) passiert hat, dieser Teil der Aussparung (5) einen Durchgang (17) zwischen dem Hohlraum (9) und der Umgebung herstellt
- B) wenn der Körper (2) die Aussparung (5) umfasst:
die Dichtung (11) auf einer Seite der Aussparung (5) angeordnet ist, die von dem Abschnitt (10) des Hohlraums (9) entfernt ist, während der Entfernung der Kappe (1) von dem Körper (2) die Dichtung (11) zu der Seite der Aussparung (5) bewegt wird, die zu dem Abschnitt (10) des Hohlraums (9) weist, und, wenn die Dichtungsoberfläche (12) mindestens einen Teil der Aussparung (5) passiert hat, dieser Teil der Aussparung (5) einen Durchgang (17) zwischen dem Hohlraum (9) und der Umgebung herstellt.

2. Anordnung nach Anspruch 1, wobei die Kappe (1) die Aussparung (5) umfasst und der Körper (2) mit der Dichtung (11) versehen ist.

3. Anordnung nach Anspruch 1 oder 2, wobei die Aussparung (5) derart angeordnet ist, dass ihr Abstand zu einem offenen Ende (7) der Kappe (1) kleiner als der Abstand zu einem geschlossenen Ende (8) der Kappe (1) ist.

4. Anordnung nach mindestens einem der vorhergehenden Ansprüche, wobei der Körper (2) mindestens eine Öffnung (29, 30, 31) umfasst, die angeordnet ist, eine Verbindung zwischen der Umgebung und einem Innenraum des Körpers (2) herzustellen wenn die Kappe (1) entfernt ist, wobei, wenn die Kappe (1) angebracht ist, diese Öffnung des Körpers (2) in dem Abschnitt (10) des Hohlraums (9) angeordnet ist.

5. Anordnung nach Anspruch 4, wobei die Öffnung (29, 30, 31) derart konfiguriert ist, dass eine Verbindung mit einem inneren Bereich des Körpers (2) hergestellt wird, der außerhalb des Abschnitts (10) angeordnet ist, wobei der Abschnitt (10) und der innere Bereich einen abgedichteten Abschnitt definieren wenn die Kappe angebracht ist.

6. Anordnung nach mindestens einem der vorhergehenden Ansprüche, wobei die Entfernung der Kappe (1) von dem Körper (2) eine relative axiale Bewegung der Kappe (1) und des Körpers (2) umfasst, wobei diese Bewegung die Ausübung einer Kraft erfordert, um einen Unterdruck zu überwinden, der in dem Abschnitt erzeugt wird, und wobei die Aussparung (5) angeordnet ist, diese Kraft in einem Maße zu verringern, das von dem axialen Abstand (d) zwischen der Dichtungsoberfläche (12) und der Aussparung (5) bestimmt wird, wenn die Kappe (1) angebracht ist.

7. Anordnung nach mindestens einem der vorhergehenden Ansprüche, wobei die Aussparung (5) durch mindestens zwei Seitenflächen mit unterschiedlichen Neigungen in Bezug auf eine Hauptausdehnungsachse von dem einem aus der Kappe (1) und des Körpers (2) begrenzt ist, der die Aussparung (5) umfasst.

8. Anordnung nach Anspruch 7, wobei die Anbringung der Kappe (1) an dem Körper (2) eine Drehschließbewegung der Kappe (1) in Bezug auf den Körper (2) in einer Schließdrehrichtung umfasst und die Seitenfläche, die einen kleineren Winkel in Bezug auf die Hauptausdehnungsachse definiert, eine Drehanschlagsfläche (13) bildet, die zum Blockieren einer weiteren Schließdrehung der Kappe (1) in Bezug auf den Körper (2) durch eine Interaktion der Drehanschlagsfläche (13) mit einer entsprechenden Fläche (14) auf dem anderen aus der Kappe (1) und dem Körper (2) konfiguriert ist, wenn die Kappe (1) an dem Körper (1) angebracht ist.

9. Anordnung nach mindestens einem der vorhergehenden Ansprüche, wobei die Entfernung der Kappe (1) von dem Körper (2) eine Drehöffnungsbewegung der Kappe (1) in Bezug auf den Körper (2) in einer Öffnungsdrehrichtung umfasst und wobei, wenn die Kappe (1) angebracht ist, die Aussparung (5) derart angeordnet ist, dass der Durchgang (17) innerhalb eines Drehwinkels von weniger als 360° hergestellt wird, wenn die Kappe (1) in Bezug auf den Körper in der Öffnungsdrehrichtung gedreht wird.

10. Anordnung nach mindestens einem der vorhergehenden Ansprüche, wobei der eine aus der Kappe (1) und dem Körper (2) mehrere Aussparungen (5) umfasst, wobei die Aussparungen entlang des Umfangs angeordnet sind.

11. Anordnung nach Anspruch 10, wobei die Aussparungen (5) derart angeordnet sind, dass benachbarte Seitenflächen von unterschiedlichen benachbarten Aussparungen unterschiedliche Neigungen in Bezug auf eine Hauptausdehnungsachse von dem einem aus der Kappe (1) und dem Körper (2) aufweisen, der die Aussparungen umfasst.

12. Anordnung nach mindestens einem der vorhergehenden Ansprüche, wobei der Körper (2) ein Basisteil (3) und ein Drehteil (19) umfasst, wobei die Teile drehbar miteinander verbunden sind und wobei, wenn die Dichtung (11) auf dem Körper (2) bereitgestellt ist, die Dichtung (11) auf dem Basisteil (3) bereitgestellt ist, und wenn die Kappe (1) angebracht ist, das Drehteil (19) in dem Abschnitt (10) angeordnet ist.

13. Anordnung nach mindestens einem der vorhergehenden Ansprüche, wobei der Körper (2) ein Mundstück (29) umfasst und wobei, wenn die Kappe angebracht ist, das Mundstück (29) in dem Abschnitt des Hohlraums (9) angeordnet ist.

14. Arzneiverabreichungsvorrichtung (200), umfassend die Anordnung nach mindestens einem der vorhergehenden Ansprüche, wobei der Körper (2) einen Arzneibehälter (24) mit einer beweglichen Wand (27) umfasst und wobei die Vorrichtung (200) ein Inhalator ist.

15. Arzneiverabreichungsvorrichtung (200) nach Anspruch 14, wobei die Anordnung derart konfiguriert ist, dass während der Entfernung der Kappe (1) von dem Körper (2), insbesondere während einer relativen axialen Bewegung der Kappe (1) und des Körpers (2) eine Dosieröffnung (23) aus dem Behälter (24) gezogen wird, wodurch eine Arzneidosis (25) aus dem Behälter (24) entnommen wird und die Wand (27) des Behälters (24) dem Unterdruck ausgesetzt wird, der in dem abgedichteten Abschnitt (10) erzeugt wird, wobei ein Dosierstift die Dosieröffnung umfasst, wobei der Dosierstift in dem Körper gehalten wird.

## Revendications

1. Agencement (100) pour un dispositif d'administration de médicaments (200), l'agencement (100) comprenant :
- un capuchon (1) définissant une cavité (9) et
- un corps (2),
- l'un du capuchon (1) et du corps (2) comprenant une échancrure (5),
- l'autre du capuchon (1) et du corps (2) étant pourvu d'un joint (11), le joint (11) comprenant une surface d'étanchéité (12),
- le capuchon (1) et le corps (2) étant configurés de telle sorte que le capuchon (1) puisse être attaché de manière amovible au corps (2),
- la surface d'étanchéité (12) du joint (11), lorsque le capuchon (1) est attaché, scellant une section (10) de la cavité (9) et
- A) lorsque le capuchon (1) comprend l'échancrure (5) :
l'échancrure (5) étant disposée sur un côté du joint (11) qui est éloigné de la section (10) de la cavité (9), au cours de l'enlèvement du capuchon (1) du corps (2), l'échancrure (5) étant déplacée vers le côté du joint (11) qui fait face à la section (10) de la cavité (9), et lorsqu'au moins une partie de l'échancrure (5) a passé la surface d'étanchéité (12), cette partie de l'échancrure (5) établissant un passage (17) entre la cavité (9) et l'environnement,
- B) lorsque le corps (2) comprend l'échancrure (5) :
le joint (11) étant disposé sur un côté de l'échancrure (5) qui est éloigné de la section (10) de la cavité (9), au cours de l'enlèvement du capuchon (1) du corps (2), le joint (11) étant déplacé vers le côté de l'échancrure (5) qui fait face à la section (10) de la cavité (9) et lorsque la surface d'étanchéité (12) a passé au moins une partie de l'échancrure (5), cette partie de l'échancrure (5) établissant un passage (17) entre la cavité (9) et l'environnement.

2. Agencement selon la revendication 1, dans lequel le capuchon (1) comprend l'échancrure (5) et le corps (2) est pourvu du joint (11).

3. Agencement selon la revendication 1 ou 2, dans lequel l'échancrure (5) est disposée de telle sorte que sa distance à une extrémité ouverte (7) du capuchon (1) soit inférieure à la distance à une extrémité fermée (8) du capuchon (1).

4. Agencement selon au moins l'une des revendications précédentes, dans lequel le corps (2) comprend au moins une ouverture (29, 30, 31) qui est prévue pour établir une communication entre l'environnement et un intérieur du corps (2) lorsque le capuchon (1) est enlevé, cette ouverture du corps (2), lorsque le capuchon (1) est attaché, étant agencée dans la section (10) de la cavité (9).

5. Agencement selon la revendication 4, dans lequel l'ouverture (29, 30, 31) est configurée pour établir une communication avec une région intérieure du corps (2) qui est prévue à l'extérieur de la section (10), la section (10) et la région intérieure définissant une portion scellée lorsque le capuchon est attaché.

6. Agencement selon au moins l'une des revendications précédentes, dans lequel l'enlèvement du capuchon (1) du corps (2) comprend un mouvement axial relatif du capuchon (1) et du corps (2), lequel mouvement requiert l'application d'une force afin de surmonter une dépression générée dans la section, et l'échancrure (5) étant agencée de manière à réduire ladite force dans une mesure déterminée par la distance axiale (d) entre la surface d'étanchéité (12) et l'échancrure (5), lorsque le capuchon (1) est attaché.

7. Agencement selon au moins l'une des revendications précédentes, dans lequel l'échancrure (5) est délimitée par au moins deux faces latérales avec des inclinaisons différentes par rapport à un axe d'étendue principal de l'un du capuchon (1) ou du corps (2) comprenant l'échancrure (5).

8. Agencement selon la revendication 7, dans lequel la fixation du capuchon (1) au corps (2) comprend un mouvement de fermeture rotatif du capuchon (1) par rapport au corps (2) dans un sens de rotation de fermeture et la face latérale qui définit un plus petit angle par rapport à l'axe d'étendue principal forme une face de butée rotative (13) qui est configurée pour bloquer une rotation de fermeture supplémentaire du capuchon (1) par rapport au corps (2) par interaction entre la face de butée rotative (13) et une face correspondante (14) sur l'autre du capuchon (1) ou du corps (2) lorsque le capuchon (1) est attaché au corps (1).

9. Agencement selon au moins l'une des revendications précédentes, dans lequel l'enlèvement du capuchon (1) du corps (2) comprend un mouvement d'ouverture rotatif du capuchon (1) par rapport au corps (2) dans un sens de rotation d'ouverture et dans lequel, lorsque le capuchon (1) est attaché, l'échancrure (5) est agencée de telle sorte que le passage (17) soit établi à l'intérieur d'un angle de rotation inférieur à 360°, lorsque le capuchon (1) est tourné par rapport au corps dans le sens de rotation d'ouverture.

10. Agencement selon au moins l'une des revendications précédentes, dans lequel l'un du capuchon (1) ou du corps (2) comprend une pluralité d'échancrures (5), les échancrures étant disposées sur la circonférence.

11. Agencement selon la revendication 10, dans lequel les échancrures (5) sont agencées de telle sorte que des faces latérales adjacentes de différentes échancrures adjacentes aient des inclinaisons différentes par rapport à un axe d'étendue principal de l'un du capuchon (1) ou du corps (2) comprenant les échancrures.

12. Agencement selon au moins l'une des revendications précédentes, dans lequel le corps (2) comprend une partie de base (3) et une partie rotative (19), lesdites parties étant connectées de manière rotative l'une à l'autre, et dans lequel, lorsque le joint (11) est prévu sur le corps (2), le joint (11) est prévu sur la partie de base (3), et lorsque le capuchon (1) est attaché, la partie rotative (19) est agencée dans la section (10).

13. Agencement selon au moins l'une des revendications précédentes, dans lequel le corps (2) comprend un embout (29), et dans lequel, lorsque le capuchon est attaché, l'embout (29) est agencé dans la section de la cavité (9).

14. Dispositif d'administration de médicaments (200) comprenant l'agencement selon au moins l'une des revendications précédentes, dans lequel le corps (2) comprend un réservoir de médicaments (24) avec une paroi mobile (27), et dans lequel le dispositif (200) est un inhalateur.

15. Dispositif d'administration de médicaments (200) selon la revendication 14, dans lequel l'agencement est configuré de telle sorte que lors de l'enlèvement du capuchon (1) du corps (2), en particulier par un mouvement axial relatif du capuchon (1) et du corps (2), un trou de dosage (23) soit tiré du réservoir (24), une dose de médicament (25) étant extraite du réservoir (24) et la paroi (27) du réservoir (24) étant exposée à la dépression générée dans la section scellée (10), une goupille de dosage comprenant le trou de dosage, la goupille de dosage étant retenue dans le corps.
